# EUROPEAN PATENT APPLICATION

(11) **EP 4 837 553 A1**
(43) Date of publication of application: **11.11.2026**
(21) Application number: 24914737.2
(22) Date of filing: 14.05.2024
(51) Int. Cl.: A61F 13/496, A61F 13/494

(54) **WEARABLE HYGIENE PRODUCT**

(30) Priority: 05.01.2024 CN 202410027711
(71) Applicant: SHANDONG THRIVE MATERNITY AND INFANT PRODUCTS CO., LTD, Shandong 277500 (CN)
(72) Inventor: SUN, Feng, Zaozhuang, Shandong 277500 (CN); LIANG, Jinpan, Zaozhuang, Shandong 277500 (CN); WANG, Xishan, Zaozhuang, Shandong 277500 (CN)
(74) Representative: Cabinet Beau de Loménie
(86) International application number: PCT/CN2024/092978
(87) International publication number: WO 2025/145515

(57) **Abstract**

A wearable hygiene product, comprising: a surface layer (100) that comes into contact with a user's skin to absorb liquid; a first water-repellent layer (201) that is disposed on a side of the surface layer (100) that contacts the skin and covers a rear part of the surface layer (100); and at least one retaining strap (300) that is disposed between the surface layer (100) and the first water-repellent layer (201), so that the surface layer (100) and the first water-repellent layer (201) cannot fully adhere to each other. A first water-repellent layer (201) is added to isolate the surface layer (100) from skin at the back of the user's crotch, avoiding contact between the skin at the back of the crotch and urine in the soaked surface layer (100), thereby avoiding skin diseases and improving the user's experience. In addition, the retaining strap (300) can ensure that the surface layer (100) cannot fully adhere to the first water-repellent layer (201), creating a space between the surface layer (100) and the first water-repellent layer (201), thereby allowing urine to be absorbed by the surface layer (100) and keeping the first water-repellent layer (201) dry.

## Description

### Technical Field

The present disclosure relates to the field of hygiene care, and more particularly to a wearable hygiene product.

### Background Art

Disposable diapers are widely used by infants and young children, as well as patients who cannot care for themselves. A typical diaper comprises a top layer, a core, a waterproof membrane, and a bottom layer. When a diaper is worn, the top layer contacts the skin and guides urine to the core, allowing the core to absorb the urine. The top layer also covers the front, middle, and back of the crotch area.

When the surface layer and the core absorb a certain amount of urine, the user's skin is constantly in contact with the urine inside the soaked surface layer, which not only reduces the user's experience, but also may irritate the user's skin, potentially inducing eczema, which causes red papules and skin itching.

Therefore, there is a need for further improvement in the existing diapers.

The information disclosed in the background section of the present disclosure is only intended to increase an understanding of the overall background of the present disclosure, and should not be construed as acknowledging or implying in any way that such information constitutes the prior art that is already known to those skilled in the art.

### Summary of the Invention

The present disclosure aims to provide a wearable hygiene product that is added with a first water-repellent layer to isolate the surface layer from skin at the back of a user's crotch, avoiding contact between the skin at the back of the crotch and urine in the soaked surface layer, thereby avoiding skin diseases and improving the user's experience. In addition, the retaining strap can ensure that the surface layer cannot fully adhere to the first water-repellent layer, creating a space between the surface layer and the first water-repellent layer, thereby allowing urine to be absorbed by the surface layer and keeping the first water-repellent layer dry.

In order to address the abovementioned problems, the present disclosure provides a wearable hygiene product, comprising: a surface layer that comes into contact with a user's skin to absorb liquid; a first water-repellent layer that is disposed on a side of the surface layer that contacts the skin and covers a rear part of the surface layer; and at least one retaining strap that is disposed between the surface layer and the first water-repellent layer, so that the surface layer and the first water-repellent layer cannot fully adhere to each other.

Preferably, the wearable hygiene product further comprises: a second water-repellent layer that is disposed on a side of the surface layer that contacts the skin and covers a front part of the surface layer, wherein the retaining strap is also disposed between the surface layer and the second water-repellent layer, so that the surface layer and the second water-repellent layer cannot fully adhere to each other.

Preferably, the second water-repellent layer has through holes.

Preferably, the second water-repellent layer has openings.

Preferably, the retaining strap is bonded to the first water-repellent layer, and ends of the retaining strap are bonded to the surface layer.

Preferably, the retaining strap is elastic.

Preferably, two retaining straps are provided.

Preferably, the wearable hygiene product further comprises: three-dimensional guards that are disposed at side edges of the surface layer, and adhere to side edges of the first water-repellent layer.

Preferably, a ratio of a length of the first water-repellent layer to a length of the second water-repellent layer ranges from 0.1 to 30.

Preferably, the retaining strap has a free end; the free end extends from a gap between the surface layer and the first water-repellent layer, and extends to a rear part of an elastic part of the wearable hygiene product; alternatively, the free end extends from a gap between the surface layer and the second water-repellent layer, and extends to a front part of the elastic part of the wearable hygiene product; wherein a part of the retaining strap is bonded to the surface layer, and the retaining strap is bonded to the first water-repellent layer and/or the second water-repellent layer.

The wearable hygiene product according to embodiments of the present disclosure is added with a first water-repellent layer to isolate the surface layer from the skin at the back of the user's crotch, avoiding contact between the skin at the back of the crotch and urine in the soaked surface layer, thereby avoiding skin diseases and improving the user's experience. In addition, the retaining strap can ensure that the surface layer cannot fully adhere to the first water-repellent layer, creating a space between the surface layer and the first water-repellent layer, thereby allowing urine to be absorbed by the surface layer and keeping the first water-repellent layer dry.

The methods and devices of the present disclosure have other characteristics and advantages that will be apparent from the accompanying drawings and subsequent specific embodiments incorporated herein, or will be described in detail in the accompanying drawings and subsequent specific embodiments, which together serve to explain the specific principles of the present disclosure.

### Brief Description of Drawings

Figure 1 is a structural schematic diagram of a first wearable hygiene product according to an exemplary embodiment of the present disclosure;
Figure 2 is a structural schematic diagram of a second wearable hygiene product according to an exemplary embodiment of the present disclosure;
Figure 3 is a structural schematic diagram of a third wearable hygiene product according to an exemplary embodiment of the present disclosure;
Figure 4 is a structural schematic diagram of a fourth wearable hygiene product according to an exemplary embodiment of the present disclosure;
Figure 5 is a structural schematic diagram of a fifth wearable hygiene product according to an exemplary embodiment of the present disclosure;
Figure 6 is a structural schematic diagram of a sixth wearable hygiene product according to an exemplary embodiment of the present disclosure;
Figure 7 is a structural schematic diagram of a seventh wearable hygiene product according to an exemplary embodiment of the present disclosure.

### List of reference numerals:

| | | | |
|---|---|---|---|
| 100: | surface layer | | |
| 201: | first water-repellent layer | 202: | second water-repellent layer |
| 203: | through hole | 204: | opening |
| 300: | retaining strap | 301: | end |
| 302: | free end | | |
| 401: | rear part of an elastic part | 402: | front part of an elastic part. |

It should be understood that the accompanying drawings are not necessarily drawn to scale, but rather present simplified representations of various features to illustrate the basic principles of the present disclosure. The specific design features disclosed in the present disclosure, including specific dimensions, directions, positions, and shapes, will be partially determined by the specific application and usage environment.

Throughout the accompanying drawings, the same reference numerals refer to the same or equivalent parts of the present disclosure.

### Detailed Description of Embodiments

Reference will now be made in detail to various embodiments of the present disclosure, examples of which are presented in the accompanying drawings and described as follows. Although the present disclosure is described in conjunction with exemplary embodiments, it should be understood that this description is not intended to limit the present disclosure to these exemplary embodiments. On the contrary, the present disclosure is intended to cover not only these exemplary embodiments, but also various substitutions, modifications, equivalents, and other embodiments that can be included within the spirit and scope of the present disclosure as defined by the accompanying claims.

When a component is referred to as being "above" or "on" another component, the component may be in contact with the other component or there may be other intermediate components in between.

The wearable hygiene products mentioned in the embodiments of the present disclosure include, but are not limited to, baby diapers/pull-up pants, women's sleeping diapers/pants, and adult diapers/pull-up pants.

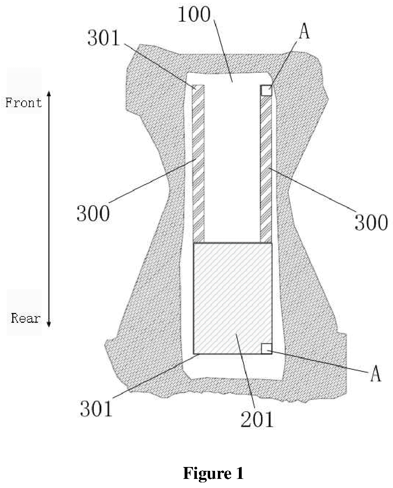

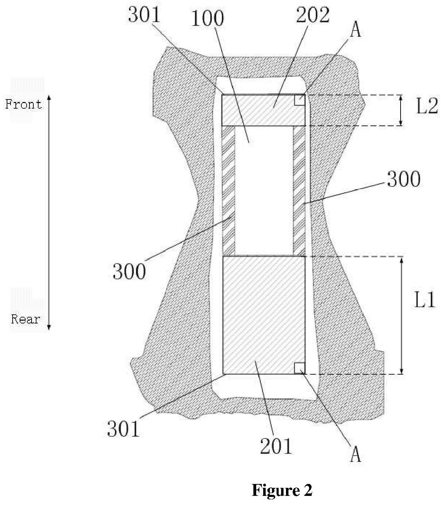

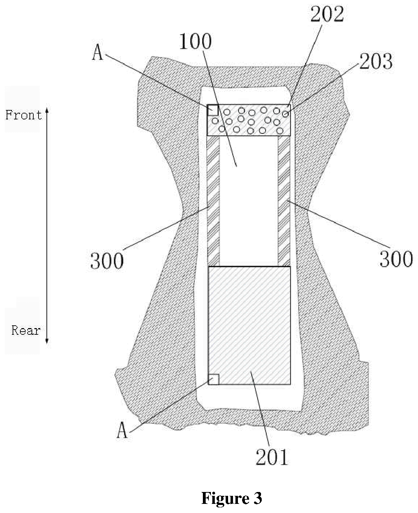

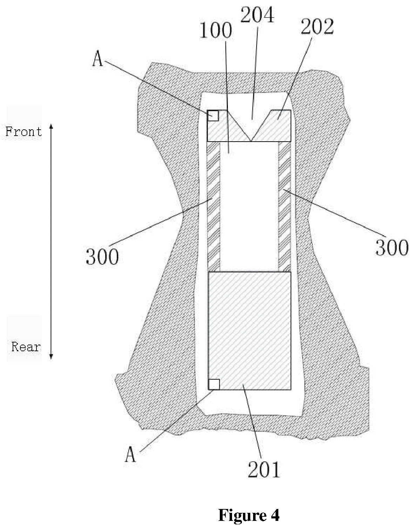

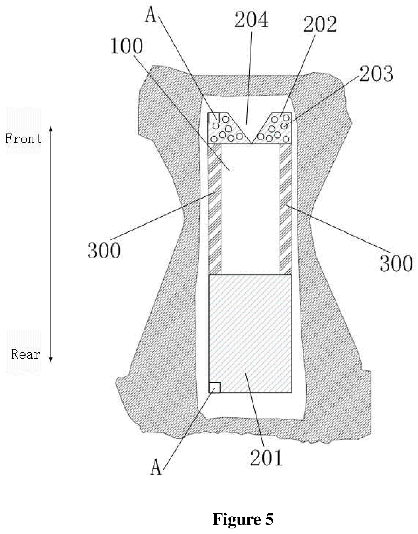

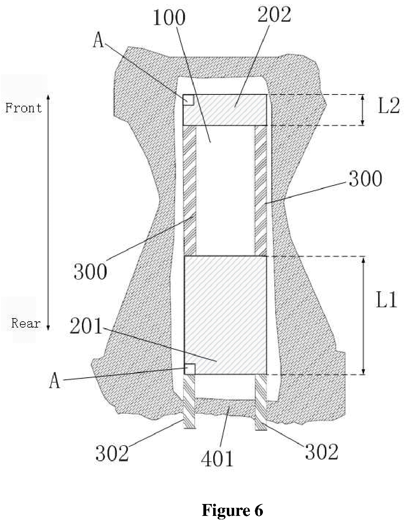

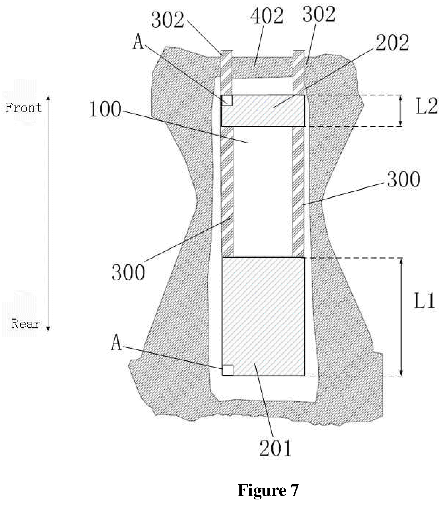

Figure 1 is a structural schematic diagram of a first wearable hygiene product according to an exemplary embodiment of the present disclosure; Figure 2 is a structural schematic diagram of a second wearable hygiene product according to an exemplary embodiment of the present disclosure; Figure 3 is a structural schematic diagram of a third wearable hygiene product according to an exemplary embodiment of the present disclosure; Figure 4 is a structural schematic diagram of a fourth wearable hygiene product according to an exemplary embodiment of the present disclosure; Figure 5 is a structural schematic diagram of a fifth wearable hygiene product according to an exemplary embodiment of the present disclosure; Figure 6 is a structural schematic diagram of a sixth wearable hygiene product according to an exemplary embodiment of the present disclosure; Figure 7 is a structural schematic diagram of a seventh wearable hygiene product according to an exemplary embodiment of the present disclosure.

Figures 1 to 7 are structural schematic diagrams of the unfolded wearable hygiene products, wherein a first water-repellent layer 201 corresponds to a rear part of a surface layer 100, and a second water-repellent layer 202 corresponds to a front part of the surface layer 100.

When a wearable hygiene product is worn, the surface layer comes into contact with the skin and wraps around the front, middle, and back parts of the crotch of human body.

The wearable hygiene products of the present disclosure will be described below with reference to Figures 1 to 7.

### Example 1

As shown in Figure 1, the wearable hygiene product comprises: a surface layer 100, a first water-repellent layer 201, and two retaining straps 300.

The surface layer 100 comes into contact with a user's skin to absorb liquid.

The first water-repellent layer 201 is disposed on a side of the surface layer 100 that contacts the skin and covers the rear part of the surface layer 100.

The retaining straps 300 are disposed between the surface layer 100 and the first water-repellent layer 201, so that the surface layer 100 and the first water-repellent layer 201 cannot fully adhere to each other. Specifically, the retaining straps 300 are bonded to the first water-repellent layer 201, and ends 301 of the retaining strap 300 are bonded to the surface layer 100. Only part of the retaining straps 300 are bonded to the surface layer 100; that is, only the ends 301 are bonded to the surface layer 100.

The wearable hygiene product according to embodiments of the present disclosure is added with a first water-repellent layer 201 to isolate the surface layer 100 from the skin at the back of the user's crotch, avoiding contact between the skin at the back of the crotch and urine in the soaked surface layer 100, thereby avoiding skin diseases and improving the user's experience. In addition, the retaining straps 300 can ensure that the surface layer 100 is not completely adhered to the first water-repellent layer 201, creating a space between the surface layer 100 and the first water-repellent layer 201, thereby allowing urine to be absorbed by the surface layer 100 and keeping the first water-repellent layer 201 dry.

### Example 2

As shown in Figure 2, the wearable hygiene product comprises: a surface layer 100, a first water-repellent layer 201, a second water-repellent layer 202, and two retaining straps 300.

The surface layer 100 comes into contact with a user's skin to absorb liquid.

The first water-repellent layer 201 is disposed on a side of the surface layer 100 that contacts the skin and covers the rear part of the surface layer 100.

The second water-repellent layer 202 is disposed on a side of the surface layer 100 that contacts the skin and covers the front part of the surface layer 100.

The retaining straps 300 are disposed between the surface layer 100 and the first water-repellent layer 201, and between the surface layer 100 and the second water-repellent layer 202, so that the surface layer 100 and the first water-repellent layer 201 cannot fully adhere to each other, and the surface layer 100 and the second water-repellent layer 202 cannot fully adhere to each other. Specifically, the retaining straps 300 are bonded to the first water-repellent layer 201 and the second water-repellent layer 202, and ends 301 of the retaining strap 300 are bonded to the surface layer 100. Only part of the retaining straps 300 are bonded to the surface layer 100; that is, only the ends 301 are bonded to the surface layer 100.

The wearable hygiene product according to embodiments of the present disclosure is added with a first water-repellent layer 201 and a second water-repellent layer 202 to isolate the surface layer 100 from the skin at the back and front parts of the user's crotch, avoiding contact between the skin at the back and front parts of the crotch and urine in the soaked surface layer, thereby avoiding skin diseases and improving the user's experience. In addition, the retaining straps 300 can ensure that the surface layer 100 is not completely adhered to the first water-repellent layer 201 and the second water-repellent layer 202, creating a space between the surface layer 100 and the first water-repellent layer 201, and between the surface layer 100 and the second water-repellent layer 202, thereby allowing urine to be absorbed by the surface layer 100 and keeping the first water-repellent layer 201 and the second water-repellent layer 202 dry.

### Example 3

As shown in Figure 3, the wearable hygiene product comprises: a surface layer 100, a first water-repellent layer 201, a second water-repellent layer 202, and two retaining straps 300.

The surface layer 100 comes into contact with a user's skin to absorb liquid.

The first water-repellent layer 201 is disposed on a side of the surface layer 100 that contacts the skin and covers the rear part of the surface layer 100.

The second water-repellent layer 202 is disposed on a side of the surface layer 100 that contacts the skin and covers the front part of the surface layer 100.

The retaining straps 300 are disposed between the surface layer 100 and the first water-repellent layer 201, and between the surface layer 100 and the second water-repellent layer 202, so that the surface layer 100 and the first water-repellent layer 201 cannot fully adhere to each other, and the surface layer 100 and the second water-repellent layer 202 cannot fully adhere to each other. The connection way of the retaining straps 300 is the same as that in Example 2 and will not be repeated here.

The second water-repellent layer 202 is provided with through holes 203.

The wearable hygiene product according to embodiments of the present disclosure is added with a first water-repellent layer 201 and a second water-repellent layer 202 to isolate the surface layer 100 from the skin at the back and front parts of the user's crotch, avoiding contact between the skin at the back and front parts of the crotch and urine in the soaked surface layer, thereby avoiding skin diseases and improving the user's experience. In addition, the through holes 203 are provided in the second water-repellent layer 202, allowing some urine at the front part of the crotch to flow into the surface layer 100 through the through holes 203. In addition, the retaining strap 300 can ensure that the surface layer 100 cannot fully adhere to the first water-repellent layer 201 and the second water-repellent layer 202, creating a space between the surface layer 100 and the first water-repellent layer 201, and between the surface layer 100 and the second water-repellent layer 202, thereby allowing urine to be absorbed by the surface layer 100 and keeping the first water-repellent layer 201 and the second water-repellent layer 202 dry.

### Example 4

As shown in Figure 4, the wearable hygiene product comprises: a surface layer 100, a first water-repellent layer 201, a second water-repellent layer 202, and two retaining straps 300.

The surface layer 100 comes into contact with a user's skin to absorb liquid.

The first water-repellent layer 201 is disposed on a side of the surface layer 100 that contacts the skin and covers the rear part of the surface layer 100.

The second water-repellent layer 202 is disposed on a side of the surface layer 100 that contacts the skin and covers the front part of the surface layer 100.

The retaining straps 300 are disposed between the surface layer 100 and the first water-repellent layer 201, and between the surface layer 100 and the second water-repellent layer 202, so that the surface layer 100 and the first water-repellent layer 201 cannot fully adhere to each other, and the surface layer 100 and the second water-repellent layer 202 cannot fully adhere to each other. The connection way of the retaining straps 300 are the same as that in Example 2 and will not be repeated here.

The second water-repellent layer 202 is provided with an opening 204 of triangular shape.

The wearable hygiene product according to embodiments of the present disclosure is added with a first water-repellent layer 201 and a second water-repellent layer 202 to isolate the surface layer 100 from the skin at the back and front parts of the user's crotch, avoiding contact between the skin at the back and front parts of the crotch and urine in the soaked surface layer, thereby avoiding skin diseases and improving the user's experience. In addition, the opening 204 is provided in the second water-repellent layer 202, allowing some urine at the front part of the crotch to flow into the surface layer 100 through the opening 204. In addition, the retaining straps 300 can ensure that the surface layer 100 cannot fully adhere to the first water-repellent layer 201 and the second water-repellent layer 202, creating a space between the surface layer 100 and the first water-repellent layer 201, and between the surface layer 100 and the second water-repellent layer 202, thereby allowing urine to be absorbed by the surface layer 100 and keeping the first water-repellent layer 201 and the second water-repellent layer 202 dry.

### Example 5

As shown in Figure 5, the wearable hygiene product comprises: a surface layer 100, a first water-repellent layer 201, a second water-repellent layer 202, and two retaining straps 300.

The surface layer 100 comes into contact with a user's skin to absorb liquid.

The first water-repellent layer 201 is disposed on a side of the surface layer 100 that contacts the skin and covers the rear part of the surface layer 100.

The second water-repellent layer 202 is disposed on a side of the surface layer 100 that contacts the skin and covers the front part of the surface layer 100.

The retaining straps 300 are disposed between the surface layer 100 and the first water-repellent layer 201, and between the surface layer 100 and the second water-repellent layer 202, so that the surface layer 100 and the first water-repellent layer 201 cannot fully adhere to each other, and the surface layer 100 and the second water-repellent layer 202 cannot fully adhere to each other. The connection way of the retaining straps 300 is the same as that in Example 2 and will not be repeated here.

The second water-repellent layer 202 is provided with an opening 204 of triangular shape, and the second water-repellent layer 202 is provided with through holes 203.

The wearable hygiene product according to embodiments of the present disclosure is added with a first water-repellent layer 201 and a second water-repellent layer 202 to isolate the surface layer 100 from the skin at the back and front parts of the user's crotch, avoiding contact between the skin at the back and front parts of the crotch and urine in the soaked surface layer, thereby avoiding skin diseases and improving the user's experience. In addition, an opening 204 and through holes 203 are provided in the second water-repellent layer 202, allowing some urine at the front part of the crotch to flow into the surface layer 100 through the opening 204 and the through holes 203. In addition, the retaining straps 300 can ensure that the surface layer 100 cannot fully adhere to the first water-repellent layer 201 and the second water-repellent layer 202, creating a space between the surface layer 100 and the first water-repellent layer 201, and between the surface layer 100 and the second water-repellent layer 202, thereby allowing urine to be absorbed by the surface layer 100 and keeping the first water-repellent layer 201 and the second water-repellent layer 202 dry.

In an exemplary embodiment, the wearable hygiene product further comprises a three-dimensional guard (not illustrated in the figures), which is provided at side edges of the surface layer. The three-dimensional guard can prevent side leakage.

In an embodiment, a side edge of the first water-repellent layer 201 adheres to the surface layer. In another embodiment, a side edge of the first water-repellent layer 201 adheres to the three-dimensional guard.

In an embodiment, a side edge of the second water-repellent layer 202 adheres to the surface layer. In another embodiment, a side edge of the second water-repellent layer 202 adheres to the three-dimensional guard.

In an exemplary embodiment, the material of the surface layer 100 may be hot-air nonwoven fabric/spunbond nonwoven fabric/spunlace nonwoven fabric, perforated film.

In an exemplary embodiment, the material of the first water-repellent layer 201 may be an SMS-type water-repellent spunmelt nonwoven fabric, an SSS-type water-repellent spunbond nonwoven fabric, a waterproof membrane, or the like.

In an exemplary embodiment, the material of the second water-repellent layer 202 may be an SMS-type water-repellent spunmelt nonwoven fabric, an SSS-type water-repellent spunbond nonwoven fabric, a waterproof membrane, or the like.

In an exemplary embodiment, the material of the retaining strap 300 may be spandex yarn, elastic film, elastic nonwoven fabric, or the like.

In an exemplary embodiment, as shown in Figure 2, the ratio of a length L1 of the first water-repellent layer 201 to a length L2 of the second water-repellent layer 202 ranges from 0.1 to 30.

When the wearable hygiene product is worn, the rear part 401 of the elastic part corresponds to the back of the waist of the human body, and the front part 402 of the elastic part corresponds to the front of the waist of the human body.

In the illustrated embodiment, the number of the retaining straps 300 is set to 2, but its number can be adjusted as needed, for example, it can be set to any number from 1 to 5.

In an exemplary embodiment, the retaining strap 300 has elasticity.

In an exemplary embodiment, as shown in Figure 6, the retaining strap 300 has a free end 302 at one end, which extends from the gap between the surface layer 100 and the first water-repellent layer 201, and extends to the rear part 401 of the elastic part of the wearable hygiene product. The retaining straps 300 may function as elastic bands. Stretching the ends of the retaining straps 300 can adjust the tightness of the retaining straps 300 and reduce the adhesion between the first water-repellent layer 201 and the surface layer 100.

In an exemplary embodiment, as shown in Figure 7, the retaining strap 300 have a free end 302 at one end, which extends from the gap between the surface layer 100 and the second water-repellent layer 202, and extends to the front part 402 of the elastic part of the wearable hygiene product. The retaining strap 300 may function as elastic bands. Stretching the ends of the retaining straps 300 can adjust the tightness of the retaining straps 300 and reduce the adhesion between the second water-repellent layer 202 and the surface layer 100.

In the embodiments of Figure 6 and Figure 7, part of the retaining straps 300 (the part corresponding to Area A in Figures 6 and 7) is bonded to the surface layer 100, and the retaining straps 300 are bonded to the first water-repellent layer 201 and the second water-repellent layer 202.

In the embodiments of Figures 1 to 7, the only place where the retaining strap 300 is bonded to the surface layer 100 is Area A, and the areas outside Area A of the retaining strap 300 are not bonded to the surface layer 100. When the wearable hygiene product is worn on a person, the retaining straps 300 make contact with the surface layer 100 solely at Area A, and the areas outside Area A of the retaining straps 300 are not in contact with the surface layer 100.

In the embodiments of Figures 1 to 7, the position of Area A can be finely adjusted at the end of the retaining strap 300 according to actual design requirements.

In the embodiments of Figures 1 to 7, the size, shape, and quantity of Area A can be adjusted according to actual design requirements.

In an exemplary embodiment, as shown in Figures 3 and 5, the shape of the through hole 203 is circular. In other embodiments, the shape of the through hole 203 is D-shaped or other shapes.

In an exemplary embodiment, the wearable hygiene product of the present disclosure further comprises a core, a waterproof membrane, and a bottom layer.

That is, in terms of the layered structure, the wearable hygiene product sequentially comprises a water-repellent layer, a surface layer, a core, a waterproof membrane, and a bottom layer from the inside out.

For convenience of interpretation and precise definition of the appended claims, the terms "up", "down", "in", "out", "above", "below", "on", "under", "upward", "downward", "front", "back", "rear", "inside", "outside", "inward", "outward", "internal", "external", "inner", "outer", "forwards", "backwards" are used to describe the features of the exemplary embodiments with reference to the positions of these features shown in the accompanying drawings.

The foregoing description of the specific exemplary embodiments of the present disclosure is for illustrative and descriptive purposes. It is not intended to be exhaustive, nor is it intended to limit the invention to the precise forms disclosed. Obviously, many changes and variations are possible based on the above teachings. The purpose of selecting and describing exemplary embodiments is to explain the specific principles and practical applications of the present disclosure, so that others skilled in the art can implement and utilize various exemplary embodiments of the present disclosure, as well as various alternatives and modifications. The scope of the present disclosure is intended to be limited by the appended claims and their equivalent forms.

## Claims

1. A wearable hygiene product, **characterized in** comprising:
a surface layer that comes into contact with a user's skin to absorb liquid;
a first water-repellent layer that is disposed on a side of the surface layer that contacts the skin and covers a rear part of the surface layer; and
at least one retaining strap that is disposed between the surface layer and the first water-repellent layer, so that the surface layer and the first water-repellent layer cannot fully adhere to each other.

2. The wearable hygiene product according to Claim 1, **characterized in** further comprising:
a second water-repellent layer that is disposed on a side of the surface layer that contacts the skin and covers a front part of the surface layer, wherein the retaining strap is also disposed between the surface layer and the second water-repellent layer, so that the surface layer and the second water-repellent layer cannot fully adhere to each other.

3. The wearable hygiene product according to Claim 2, **characterized in that** the second water-repellent layer has through holes.

4. The wearable hygiene product according to Claim 2 or 3, **characterized in that** the second water-repellent layer has openings.

5. The wearable hygiene product according to Claim 3, **characterized in that** the retaining strap is bonded to the first water-repellent layer, and ends of the retaining strap are bonded to the surface layer.

6. The wearable hygiene product according to Claim 1, **characterized in that** the retaining strap is elastic.

7. The wearable hygiene product according to Claim 2, **characterized in that** two retaining straps are provided.

8. The wearable hygiene product according to Claim 1, **characterized in** further comprising:
three-dimensional guards that are disposed at side edges of the surface layer, and adhere to the side edges of the first water-repellent layer.

9. The wearable hygiene product according to Claim 2, **characterized in that** a ratio of a length of the first water-repellent layer to a length of the second water-repellent layer ranges from 0.1 to 30.

10. The wearable hygiene product according to Claim 2, **characterized in that** the retaining strap has a free end;
the free end extends from a gap between the surface layer and the first water-repellent layer, and extends to a rear part of an elastic part of the wearable hygiene product; or
the free end extends from a gap between the surface layer and the second water-repellent layer, and extends to a front part of the elastic part of the wearable hygiene product;
wherein a part of the retaining strap is bonded to the surface layer, and the retaining strap is bonded to the first water-repellent layer and/or the second water-repellent layer.
